# EUROPÄISCHE PATENTSCHRIFT

(11) **EP 1 583 733 B1**
(45) Veröffentlichungstag und Bekanntmachung des Hinweises auf die Patenterteilung: **24.04.2013**
(21) Anmeldenummer: 03775379.5
(22) Anmeldetag: 21.11.2003
(51) Int. Cl.: C07C 67/03, C07C 69/54, B01J 21/06

(54) **VERBESSERTES VERFAHREN ZUR KONTINUIERLICHEN HERSTELLUNG VON ALKYL(METH)ACRYLATEN MIT MEHRFACHER KATALYSATORREZYKLIERUNG**
IMPROVED METHOD FOR THE CONTINUOUS PRODUCTION OF ALKYL(METH)ACRYLATES WITH MULTIPLE CATALYST RECYCLING
AMELIORATION APPORTEE A UN PROCEDE DE PRODUCTION EN CONTINU D'ALKYL(METH)ACRYLATES AVEC RECYCLAGE MULTIPLE DU CATALYSEUR

(30) Priorität: 13.01.2003 DE 10301007
(43) Veröffentlichungstag der Anmeldung: 12.10.2005
(73) Patentinhaber: Evonik Röhm GmbH, 64293 Darmstadt (DE)
(72) Erfinder: ACKERMAN, Jochen, 64367 Mühltal (DE); HILTNER, Horst, 64646 Heppenheim (DE); SIEGERT, Hermann, 64342 Seeheim-Jugenheim (DE)
(86) Internationale Anmeldenummer: PCT/EP2003/013060
(87) Internationale Veröffentlichungsnummer: WO 2004/063140

(56) Entgegenhaltungen:
- DE-A- 2 319 688
- DE-A- 10 026 644

## Beschreibung

### Gebiet der Erfindung

Die vorliegende Erfindung betrifft ein weiteres verbessertes kontinuierliches Verfahren zur Herstellung von Alkyl(meth)acrylaten (C) durch kontinuierliche Umesterung von Methyl(meth)acrylat (A) mit Alkoholen (B) unter Freisetzung von Methanol (D) gemäß folgender Reaktionsgleichung:

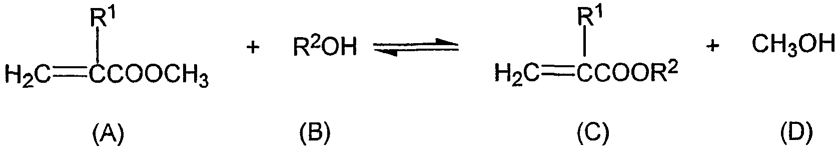

R¹ = H, CH₃

worin R² für einen linearen, verzweigten oder cyclischen Alkylrest oder Arylrest mit 2 bis 12 C-Atomen steht. Als Gruppe R² kommt beispielsweise die Ethyl-, n-Propyl-, iso-Propyl-, Allyl-, n-Butyl-, 1-Methyl-propyl-, 2-Methyl-propyl-, tert.-Butyl-, n-Pentyl-, 1-Methyl-butyl-, 2-Methyl-butyl-, 3-Methyl-butyl-, 2,2-Dimethylpropyl-, n-Hexyl-, 1-Methyl-pentyl-, 2-Methyl-pentyl-, 3-Methyl-pentyl-, 4-Methyl-pentyl-, 1,1-Dimethyl-butyl-, 2,2-Dimethyl-butyl-, 3,3-Dimethyl-butyl-, 1,2-Dimethyl-butyl-, n-Heptyl-, 1-Methyl-hexyl-, 2-Methyl-hexyl-, 3-Methyl-hexyl-, 4-Methyl-hexyl-, 1,2-Dimethyl-pentyl-, 1,3-Dimethyl-pentyl-, 1,1-Dimethyl-pentyl-, 1,1,2,2-Tetramethyl-propyl-, Benzyl-, n-Octyl-, 2-Ethylhexyl-, n-Nonyl-, 1-Methyl-octyl-, 2-Methyl-octyl-, n-Decyl-, n-Undecyl-, 1-Methyl-decyl-, 2-Methyl-decyl-, n-Dodecyl-, 2,4-Diethyl-octyl-, Cyclopentyl-, Cyclohexyl-, 4-tert.-Butyl-cyclohexyl-, Cycloheptyl-, Cyclododecyl-, 2-(Dimethylamino)-ethyl-, 3-(Dimethylamino)-propyl-, 4-(Dimethylamino)-butyl-, 5-(Dimethylamino)-pentyl-, 6-(Dimethylamino)-hexyl-, 8-(Dimethylamino)-octyl-, 10-(Dimethylamino)-decyl-, 12-(Dimethylamino)-dodecyl-, 2-(Diethylamino)-ethyl-, 3-(Diethylamino)-propyl-, 4-(Diethylamino)-butyl-, 5-(Diethylamino)-pentyl-, 6-(Diethylamino)-hexyl-, 8-(Diethylamino)-octyl-, 10-(Diethylamino)-decyl-, 12-(Diethylamino)-dodecyl-, 2-(Di-(iso-propyl)-amino)-ethyl-, 3-(Di-(iso-propyt)-amino)-propyl-, 4-(Di-(iso-propyl)-amino)-butyl-, 5-(Di-(iso-propyl)-amino)-pentyl-, 6-Di-(iso-propyl)-amino)-hexyl-, 8-(Di-(iso-propyl)-amino)-octyl-, 10-(Di-(iso-propyl)-amino)-decyl-, 12-(Di-(iso-propyl)-amino)-dodecyl-, 2-(Dibutylamino)-ethyl-, 3-(Dibutylamino)-propyl-, 4-(Dibutylamino)-butyl-, 5-(Dibutylamino)-pentyl-, 6-(Dibutylamino)-hexyl-, 8-(Dibutylamino)-octyl-, 10-(Dibutylamino)-decyl-, 12-(Dibutylamino)-dodecyl-, 2-(Dihexylamino)-ethyl-, 3-(Dihexylamino)-propyl-, 4-(Dihexylamino)-butyl-, 5-(Dihexylamino)-pentyl-, 6-(Dihexylamino)-hexyl-, 8-(Dihexylamino)-octyl-, 10-(Dihexylamino)-decyl-, 12-(Dihexylamino)-dodecyl-, 2-(Methyl-ethyl-amino)-ethyl-, 2-(Methyl-propyl-amino)-ethyl-, 2-(Methyl-iso-propyl-amino)- ethyl-, 2-(Methyl-butyl-amino)-ethyl-, 2-(Methyl-hexyl-amino)-ethyl-, 2-(Methyl-octyl-amino)-ethyl-, 2-(Ethyl-propyl-amino)-ethyl-, 2-(Ethyl-iso-propyl-amino)-ethyl-, 2-(Ethyl-butyl-amino)-ethyl-, 2-(Ethyl-hexyl-amino)-ethyl-, 2-(Ethyl-octyl-amino)-ethyl-, 3-(Methyl-ethyl-amino)-propyl-, 3-(Methyl-propyl-amino)-propyl-, 3-(Methyl-iso-propyl-amino)-propyl-, 3-(Methyl-butyl-amino)-propyl-, 3-(Methyl-hexyl-amino)-propyl-, 3-(Methyl-octyl-amino)-propyl-, 3-(Ethyl-propyl-amino)-propyl-, 3-(Ethyl-iso-propyl-amino)-propyl-, 3-(Ethyl-butyl-amino)-propyl-, 3-(Ethyl-hexyl-amino)-propyl-, 3-(Ethyl-octyl-amino)-propyl-, 4-(Methyl-ethyl-amino)-butyl-, 4-(Methyl-propyl-amino)-butyl-, 4-(Methyl-iso-propyl-amino)-butyl-, 4-(Methyl-butyl-amino)-butyl-, 4-(Methyl-hexyl-amino)-butyl-, 4-(Methyl-octyl-amino)-butyl-, 4-(Ethyl-propyl-amino)-butyl-, 4-(Ethyl-iso-propyl-amino)-butyl-, 4-(Ethyl-butyl-amino)-butyl-, 4-(Ethyl-hexyl-amino)-butyl-, 4-(Ethyl-octyl-amino)-butyl-, 2-(N-Piperidinyl)-ethyl-, 3-(N-Piperidinyl)-propyl-, 4-(N-Piperidinyl)-butyl-, 5-(N-Piperidinyl)-pentyl-, 6-(N-Piperidinyl)-hexyl-, 8-(N-Piperidinyl)-octyl-, 10-(N-Piperidinyl)-decyl-, 12-(N-Piperidinyl)-dodecyl-, 2-(N-Pyrrolidinyl)-ethyl-, 3-(N-Pyrrolidinyl)-propyl-, 4-(N-Pyrrolidinyl)-butyl-, 5-(N-Pyrrolidinyl)-pentyl-, 6-(N-Pyrrolidinyl)-hexyl-, 8-(N-Pyrrolidinyl)-octyl-, 10-(N-Pyrrolidinyl)-decyl-, 12-(N-Pyrrolidinyl)-dodecyl-, 2-(N-Morpholino)-ethyl-, 3-(N-Morpholino)-propyl-, 4-(N-Morpholino)-butyl-, 5-(N-Morpholino)-pentyl-, 6-(N-Morpholino)-hexyl-, 8-(N-Morpholino)-octyl-, 10-(N-Morpholino)-decyl-, 12-(N-Morpholino)-dodecyl-, 2-(N'-Methyl-N-Piperazinyl)-ethyl-, 3-(N'-Methyl-N-Piperazinyl)-propyl-, 4-(N'-Methyl-N-Piperazinyl)-butyl-, 5-(N'-Methyl-N-Piperazinyl)-pentyl-, 6-(N'-Methyl-N-Piperazinyl)-hexyl-, 8-(N'-Methyl-N-Piperazinyl)-octyl-, 10-(N'-Methyl-N-Piperazinyl)-decyl-, 12-(N'-Methyl-N-Piperazinyl)-dodecyl-, 2-(N'-Ethyl-N-Piperazinyl)-ethyl-, 3-(N'-Ethyl-N-Piperazinyl)-propyl-, 4-(N'-Ethyl-N-Piperazinyl)-butyl-, 5-(N'-Ethyl-N-Piperazinyl)-pentyl-, 6-(N'-Ethyl-N-Piperazinyl)-hexyl-, 8-(N'-Ethyl-N-Piperazinyl)-octyl-, 10-(N'-Ethyl-N-Piperazinyl)-decyl-, 12-(N'-Ethyl-N-Piperazinyl)-dodecyl-, 2-(N'-iso-Propyl-N-Piperazinyl)-ethyl-, 3-(N'-iso-Propyl-N-Piperazinyl)-propyl-, 4-(N'-iso-Propyl-N-Piperazinyl)-butyl-, 5-(N'-iso-Propyl-N-Piperazinyl)-pentyl-, 6-(N'-iso-Propyl-N-Piperazinyl)-hexyl-, 8-(N'-iso-Propyl-N-Piperazinyl)-octyl-, 10-(N'-iso-Propyl-N-Piperazinyl)-decyl-, 12-(N'-iso-Propyl-N-Piperazinyl)-dodecyl-, 3-Oxa-butyl-, 3-Oxa-pentyl-, 2,2-Dimethyl-4-oxa-pentyl-, 3,6-Dioxa-heptyl-, 3,6-Dioxa-octyl-, 3,6,9-Trioxa-decyl-, 3,6,9-Trioxaundecyl-, 4-Oxa-pentyl-, 4-Oxa-hexyl-, 4-Oxa-heptyl-, 4,8-Dioxa-nonyl-, 4,8-Dioxa-decyl-, 4,8-Dioxa-undecyl-, 5-Oxa-hexyl- oder die 5,10-Dioxa-undecyl-Gruppe in Frage.

Weiterhin kann es sich bei R²OH um ethoxylierte und/oder propoxylierte Alkohole sowie gemischt-ethoxylierte/propoxylierte Alkohole wie R⁵-(O-CH₂-CH₂)ₓ-OH oder
R⁵-(O-CH(CH₃)-CH₂)ₓ-OH beziehungsweise R⁵-(O-CH₂-CH(CH₃))ₓ-OH,
worin
R⁵ für C₁ bis C₂₀-Alkyl und
x für eine ganze Zahl zwischen 10 und 20 steht
oder um ethoxylierte und/oder propoxylierte Aminoalkohole
R³₂N(-CH₂-CH₂-O)_{y}-H oder
R³₂N(-CH(CH₃)-CH₂-O)y-H beziehungsweise R³₂N(-CH₂CH(CH₃)-O)_{y}-H,
worin y für eine ganze Zahl zwischen 1 und 4 steht,
handeln.
R³ steht für eine Alkylgruppe mit 1 - 6 Kohlenstoffatomen, wobei der Stickstoff mit den Substituenten R³ auch einen fünf- bis siebengliedrigen Ring bilden kann. Der Ring kann gegebenenfalls noch durch eine oder mehrere kurzkettige Alkylgruppen, wie beispielsweise Methyl, Ethyl oder Propyl, substituiert sein.

Im erfindungsgemäßen Verfahren werden vorzugsweise n-Butanol, iso-Butanol und 2-Ethylhexanol eingesetzt.

Die Verbesserung gegenüber dem Stand der Technik besteht darin, dass der bevorzugt verwendete homogene Katalysator Tetraalkyltitanat (Tetraalkoxytitan) durch Rezyklierung überraschenderweise mehrfach genutzt werden kann. Dadurch wird der Katalysatorverbrauch und folglich die Hilfsstoffkosten entscheidend reduziert, wodurch die Wirtschaftlichkeit des Verfahrens nochmals deutlich erhöht wird.

### Stand der Technik

Alkyl(meth)acrylate können durch Umesterung von Methyl(meth)acrylat in Anwesenheit von Katalysatoren auf verschiedene Verfahrensweisen kontinuierlich hergestellt werden (DE-A-100 26 644).

EP 0 960 877 (Elf Atochem S.A.) beschreibt ein kontinuierliches Verfahren zur Herstellung von Methacrylatestern von Dialkylaminoalkoholen. Man setzt Dialkylaminoalkohole mit im allgemeinen Methyl(meth)acrylat um und erhält das Dialkylaminoalkyl(meth)acrylat nach folgendem Verfahren:
Das Gemisch der Ausgangsstoffe (Methyl(meth)acrylat und Dialkylaminoalkohol) wird zusammen mit einem Tetraalkyltitanat als homogenem Umesterungskatalysator (beispielsweise Tetrabutyl-, Tetraethyl- oder Tetra(2-Ethylhexyl)titanat) und mindestens einem Polymerisationsinhibitor (beispielsweise Phenothiazin, tert.-Butylcatechol, Hydrochinonmonomethylether oder Hydrochinon) kontinuierlich einem Rührreaktor zugeführt, wo bei einer, Temperatur von 90 - 120 °C die Umsetzung zum Dialkylamino(meth)acrylat bei gleichzeitigem kontinuierlichem Abzug des azeotropen Methyl(meth)acrylat/ Methanol-Gemisches erfolgt. Das rohe Reaktionsgemisch (Rohester) wird einer ersten Destillationskolonne zugeführt, wobei man bei vermindertem Druck am Kopf der Destillationskolonne einen im wesentlichen katalysatorfreien Strom abzieht und im Sumpf der Destillationskolonne der Katalysator sowie wenig Dialkylaminoalkyl(meth)acrylat abgezogen werden. Der Kopfstrom der ersten Destillationskolonne wird dann einer zweiten Destillationskolonne zugeführt, bei der man unter vermindertem Druck am Kopf einen Strom von niedrigsiedenden Produkten mit wenig Dialkylaminoalkyl(meth)acrylat und im Sumpf einen Strom bestehend aus hauptsächlich Dialkylaminoalkyl(meth)acrylat sowie Polymerisationsinhibitor(en) abzieht, der einer dritten Destillationskolonne zugeführt wird. In der dritten Destillationskolonne wird unter vermindertem Druck eine Rektifikation durchgeführt, in der man am Kopf den gewünschten reinen Dialkylaminoalkyl(meth)acrylatester und im Sumpf im wesentlichen den Polymerisationsinhibitor oder die Polymerisationsinhibitoren abzieht. Der Sumpfstrom der ersten Destillationskolonne wird nach weiterer Aufreinigung mit Hilfe eines Filmverdampfers genauso in den Reaktor zurückgeführt wie der Kopfstrom aus der zweiten Destillationskolonne.

Eine detaillierte Beschreibung der Vorgehensweise zur Rezyklierung des homogenen Katalysators wird nicht angegeben. Aus der angegebenen Aufreinigung des Sumpfabstroms der ersten Destillationskolonne über einen Filmverdampfer kann man lediglich ableiten, dass das restliche Dialkylaminoalkyl(meth)acrylat vom Katalysator sowie hochsiedenden Nebenkomponenten abgetrennt und in den Reaktor rezykliert wird.

EP 0 968 995 (Mitsubishi Gas Chemical Comp.) beschreibt ein kontinuierliches Verfahren zur Herstellung von Alkyl(meth)acrylsäureestern unter Verwendung einer Reaktionskolonne. Die Umesterungsreaktion erfolgt dabei direkt in einer Destillationskolonne (d.h. Reaktor und Destillationskolonne zum Abzug des Methyl(meth)acrylat/Methanol-Azeotrops bilden einen Apparat), der die Ausgangsstoffe (Methyl(meth)acrylat und Alkohol) kontinuierlich zugeführt werden. Der notwendige Katalysator, hier ebenfalls bevorzugt eine Titanverbindung, befindet sich in der Destillationskolonne. Im Fall eines homogenen Katalysators wird der Katalysator in die Destillationskolonne kontinuierlich eindosiert. Die Verwendung von homogenen Katalysatoren in einer Destillationskolonne führt jedoch aufgrund eines Spüleffektes durch den flüssigen Rücklauf in der Destillationskolonne zu einem erhöhten Katalysatorbedarf sowie bei Auftreten eines Katalysatorfeststoffniederschlags zur Verschmutzung der Kolonneneinbauten. Im Fall eines heterogenen Katalysators befindet sich der Katalysator in der Reaktionskolonne. Die Positionierung des Katalysators in der Destillationskolonne ist allerdings nachteilig, weil in der Destillationskolonne dann ein erhöhter Druckverlust auftritt und zusätzlich für die regelmäßige Reinigung der Destillationskolonne ein sehr hoher Aufwand betrieben werden muss. Weiterhin können heterogene Katalysatoren beispielsweise infolge unerwünschter Polymerisation desaktivieren.

Angaben zur Vorgehensweise zur Rezyklierung eines homogenen Katalysators in die Reaktionskolonne nach vorheriger Abtrennung vom Rohester werden keine gemacht.

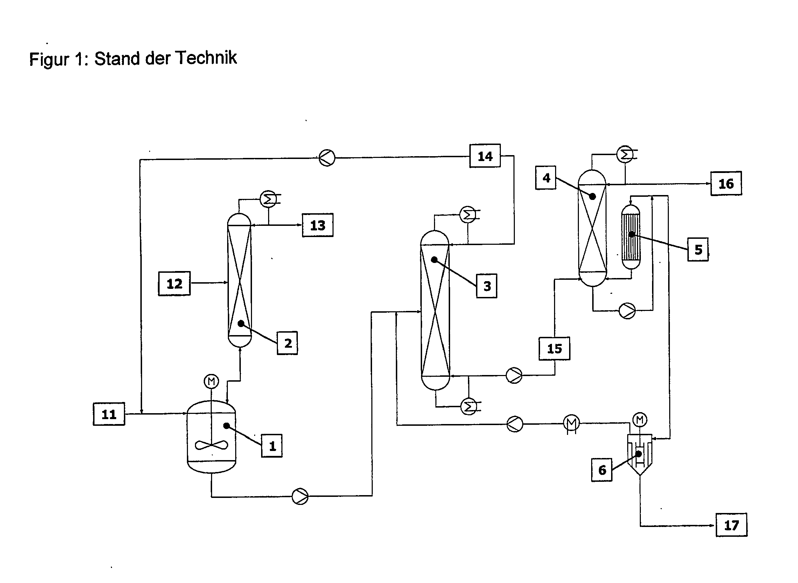

Gegenüber den zuvor genannten Verfahren beschreibt die deutsche Patentanmeldung Nr. 102 00 171.5 ein deutlich verbessertes kontinuierliches Verfahren zur Herstellung von Alkyl(meth)acrylaten durch Umesterung von Methyl(meth)acrylat mit gegenüber Methanol hochsiedenden Alkoholen (vgl. Figur 1).

Die Edukte Methyl(meth)acrylat (MMA, 11) und Alkohol (12) werden kontinuierlich einem geeigneten Reaktionsapparat (1) zugeführt, wobei sowohl ein einzelner Reaktionsbehälter als auch eine Kaskade von mehreren hintereinander geschaltenen Reaktionsbehältern eingesetzt werden kann. Es ist sinnvoll, dass alle Reaktionsbehälter einen Brüdenabzug zur Azeotrop-Destillationskolonne (2) zur Entfernung des bei der Reaktion frei werdenden Methanols besitzen. Der bevorzugt verwendete homogene Katalysator Tetraalkyltitanat (der Tetraalkoxytitan-Gehalt in Bezug auf eingesetztes MMA beträgt bevorzugt 0,2 - 0,5 Gew.-%) wird wie der/die Polymerisationsinhibitorien kontinuierlich in den Reaktionsapparat (1) zudosiert. Als Umesterungskatalysatoren können aber auch alle aus dem Stand der Technik bekannten Umesterungskatalysatoren eingesetzt werden. Als Polymerisationsinhibitor kommt beispielsweise Hydrochinonmonomethylether in Verbindung mit Sauerstoff in Frage. Da der eingesetzte Alkohol Wasser enthalten kann (die Menge an Wasser im eingesetzten Alkohol liegt im Falle von n-Butanol zwischen 0,05 - 0,005 Gew.-%), wird der Alkohol vor dem Eintreten in den Reaktionsapparat bevorzugt über die Azeotrop-Kolonne (2) destillativ entwässert. Dabei wird das im Alkohol enthaltene Wasser über Kopf abgezogen. Zur Vermeidung einer Verunreinigung des Methanol/MMA-Azeotrops (13) mit dem eingesetzten Alkohol erfolgt die Aufgabe des Alkohols bevorzugt im unteren Teil der Destillationskolonne (2). Der eingesetzte Alkohol kann aber auch durch eine vorgeschaltete Entwässerungs-Destillationskolonne, durch Behandlung mit einem Entwässerungsmittel, wie beispielsweise ein Molekularsieb, oder durch ein Membrantrennverfahren, wie beispielsweise eine Pervaporation, entwässert werden. Die Entwässerung ist daher bedeutsam, da das im Alkohol enthaltene Wasser zur irreversiblen Schädigung des Katalysators (z.B. Tetraalkyltitanat) im Reaktor führen kann. Durch diesen Entwässerungsschritt vermeidet man die Hydrolyse des Katalysators und die damit entstehenden Kosten durch erhöhte Katalysatoreinsatzmengen und durch Probleme mit Feststoffniederschlägen. Die Umsetzung erfolgt im Reaktionsapparat bei einer Temperatur im Bereich zwischen 80 und 160 °C. Bevorzugt ist der Temperaturbereich zwischen 110 und 135 °C. Zur positiven Beeinflussung des Reaktionsgleichgewichtes wird das bei der Reaktion frei werdende Methanol über die Destillationskolonne (2) aus dem Reaktionsgemisch als Azeotrop mit MMA abgezogen (13). Die Reaktionsmischung, welche größtenteils aus dem Produkt-Alkyl(meth)acrylat, nicht umgesetztem MMA und Alkohol sowie geringen Mengen Methanol, dem Katalysator, den Polymerisationsinhibitoren und einem sehr geringen Anteil an Nebenprodukten besteht, wird nach ca. 0,5 - 3 Stunden Reaktorverweilzeit (bevorzugt ist eine Verweilzeit von 0,75 - 1,5 Stunden) einer kontinuierlich betriebenen Niedrigsieder-Destillationskolonne (3) zugeführt. Dort erfolgt bei vermindertem Druck, bevorzugt im Bereich von 20 - 200 mbar, die Abtrennung der in Bezug auf den Produktester niedrigsiedenden Komponenten, vorwiegend Methanol, MMA und nicht umgesetzter Edukt-Alkohol. Diese werden über den Kopf der Destillationskolonne abgezogen und in den Reaktorbereich zurückgeführt (14). Durch diesen Kreislaufstrom wird garantiert, dass, bezogen auf den gesamten Prozess, praktisch vollständiger Umsatz in Bezug auf die Edukte MMA und Alkohol vorliegt. Der im Sumpf der Destillationskolonne (3) anfallende mit Katalysator, Polymerisationsinhibitor und hochsiedenden Nebenprodukten noch verunreinigte Roh-Ester (15) enthält vorzugsweise > 98 Gew.-% Produktester und wird zur Aufarbeitung einer weiteren Vakuumdestillationsstufe (4, 5), welche im bevorzugten Druckbereich zwischen 20 und 200 mbar arbeitet, kontinuierlich zugeführt. Hier erfolgt die kontinuierliche destillative Abtrennung des hochreinen Produktesters als Kopfprodukt (16). Verwendet man zur Abtrennung des Katalysators und der Polymerisationsinhibitoren sowie der hochsiedenden Nebenprodukte aus dem Roh-Ester (15) eine konventionelle Vakuumdestillationskolonne wie im Stand der Technik beschrieben, so kommt es im Sumpf der Kolonne infolge unzulässig hoher thermischer Belastung zu einer Zersetzung des Katalysators und damit zu einer Freisetzung des Eduktalkohols und teilweise auch zu einer Bildung von Ethern des Eduktalkohols. Beide Verbindungen (Eduktalkohol und Ether des Eduktalkohols) stellen in Bezug auf den Produktester niedrigsiedende Komponenten dar und gelangen somit als Verunreinigung in den Produktester, wodurch die Produktqualität deutlich gesenkt wird. Dieses Problem kann dadurch gelöst werden, dass man zur Abtrennung des Produktesters vom Katalysator und den Polymersiationsinhibitoren sowie den hochsiedenden Nebenprodukten einen Apparat mit schonender Filmverdampfung (5) einsetzt. Als geeignete Apparate sind hierfür Fallfilm-, Dünnschicht- und Kurzwegverdampfer bekannt. Zum Erreichen der höchsten Produktester-Reinheit (Produktester > 99,9 Gew.-%, Alkohol < 120 ppm, MMA < 10 ppm, Ether < 5 ppm, Farbzahl (Apha) < 1) dient eine weitere, nachgeschaltete Hochsieder-Destillationsstufe (4). Hierbei hat ein einzelner Apparat mit Filmverdampfung aber den Nachteil, eine unzureichende Reinigungsleistung zu bieten, so dass hochsiedende Nebenprodukte mit in den Rein-Produktester (16) gelangen. Dieses Problem wird dadurch gelöst, dass zur Abtrennung der hochsiedenden Nebenprodukte vom Rein-Produktester oberhalb des Apparates mit Filmverdampfung eine Vakuumrektifikationskolonne (4) angeordnet wird. Nach Abtrennung des Katalysators und der Polymerisationsinhibitoren sowie der hochsiedenden Nebenprodukte aus dem Roh-Ester verbleibt ein gewisser Anteil an Produktester im Sumpfprodukt, damit der Sumpfabstrom noch gut fließ- und förderfähig bleibt. Um bei der Ausschleusung von Katalysator, Polymerisationsinhibitoren und hochsiedenden Nebenprodukten (17) den Verlust an Produktester zu minimieren, sollte eine Vakuum-Eindampfstufe, welche im bevorzugten Druckbereich von 20 - 200 mbar arbeitet, nachgeschaltet werden (6). Für diese Aufgabe kommt wiederum ein Apparat mit Filmverdampfung in Frage. Als geeignete Apparate sind hierfür wiederum Fallfilm-, Dünnschicht- und Kurzwegverdampfer bekannt. Der an der Eindampfstufe über Kopf abgeführte Produktester erfüllt aufgrund eines unzulässigen Gehaltes an hochsiedenden Komponenten nicht die geforderte Spezifikation für den Rein-Produktester. Weiterhin enthält er aufgrund der thermischen Zersetzung des Katalysators noch Eduktalkohol und teilweise auch Ether des Eduktalkohols. Aus diesen Gründen kann der Destillatstrom mit dem Ziel, den darin enthaltenen Produktester zu gewinnen, nicht direkt in die Hochsieder-Destillationskolonne (4) zurückgeführt werden, vielmehr muss die Rückführung zum Reaktionsapparat (1) oder vorteilhaft zur Niedrigsieder-Destillationskolonne (3) erfolgen, um die Niedrigsieder vor der ersten Eindampfstufe (5) abzutrennen.

Das Verfahren besitzt den Nachteil, dass der mehrfach thermisch beanspruchte und somit möglicherweise geschädigte, teilweise inaktive homogene Katalysator nach seiner Abtrennung vom Produktester zusammen mit den Polymerisationsinhibitoren und hochsiedenden Nebenprodukten vollständig ausgeschleust wird und durch Rezyklierung nicht wiederverwendet wird. Dies führt aufgrund des relativ hohen Katalysatorpreises zu erhöhten Hilfsstoffkosten.

DE 101 27 939 (BASF) beschreibt ein kontinuierliches Umesterungsverfahren. Als eine zu lösende Teilaufgabe wird formuliert, dass der Katalysator problemlos wiederverwendet werden soll.

Der vorliegenden Erfindung lag nun die Aufgabe zugrunde, ein Verfahren zu entwickeln, das frei ist von den genannten Schwächen und folgende Kriterien erfüllt:
1. Die Ausgangsstoffe (Katalysator, Stabilisator, niederer (Meth)acrylsäureester) sollen kostengünstig, problemlos handhabbar und in technischer Menge verfügbar sein.
2. Der Katalysator soll bei erhöhter Termperatur und in Gegenwart geringer Wassermengen stabil sein.
3. Der Aktivitätsverlust des Katalysators soll gering sein und er soll problemlos wiederverwendet werden können.
4. Es soll kein systemfremder Alkohol über den Umesterungskatalysator in die Umesterung eingeschleust werden.
5. Hohe Laufzeiten der Anlage, d. h. möglichst geringe Polymerisationsprobleme und Verwendung möglichst weniger reparaturanfälliger Apparate.
6. Direkte Wiederverwendung bzw. Verwertung des anfallenden Gemisches beziehungsweise Azeotrops aus niederem Alkanol und dem entsprechenden Ester.
7. Weitgehende Rückgewinnung der restlichen Wertprodukte aus den Abfallströmen und aus den Nebenprodukten.
8. Die Umesterung soll vorzugsweise kontinuierlich betrieben werden.
9. Der Zielester soll eine hohe Reinheit (mindestens 99,9 %) aufweisen und im Falle der Herstellung von Dialkylaminomethyl(meth)acrylaten soll die Bildung von Ethylenglykoldi(meth)acrylat und Vinyloxyethyl(meth)acrylat verringert werden, möglichst auf unter 100 ppm.
10. Die Abfallmengen sollen möglichst gering und gut handhabbar sein.
11. Umsatz und Ausbeute sollen hoch sein (> 95 %).
12. Die Verweilzeiten sollen gering sein.
13. Das Gesamtverfahren soll technisch einfach und wirtschaftlich sein.

Diese Aufgabe wird zwar formuliert, aber in der Beschreibung finden sich keine Hinweise auf eine gezielte Rückführung des aktiven oder teilaktiven Katalysators. Weiter fehlen Angaben über die zahlenmäßigen Verhältnisse bei der Rückführung des Katalysators und Angaben zu eventuellen Änderungen der Produktqualität bei Verwendung eines ganz oder teilweise rezyklierten Umesterungskatalysators.

### Aufgabe

Aufgabe der vorliegenden Erfindung ist es, ein kontinuierliches Verfahren zur Umesterung von (Meth)acrylsäuremethylester (A) mit gegenüber Methanol (D) höhersiedenden Alkoholen (B) zur Verfügung zu stellen, bei dem der eingesetzte homogene Katalysator durch partielle Rezyklierung mehrfach verwendet werden kann. Unter (Meth)acrylsäureestern oder Alkyl(meth)acrylaten werden im folgenden Ester und Derivate der Acrylsäure und der Methacrylsäure verstanden. Weiterhin soll durch das neue Verfahren ein Produkt zur Verfügung gestellt werden, das in der Qualität besser ist als die bisher auf dem Markt befindlichen. Ferner sollen mit dem neuen Verfahren Alkyl(meth)acrylate mit möglichst wenig Aufwand und energetisch günstiger und mit geringerem Hilfsstoffeinsatz (d.h. kostengünstiger) hergestellt werden. Gelöst werden diese sowie weitere im einzelnen nicht näher ausgeführte jedoch aus der einleitenden Erörterung des Standes der Technik ohne weiteres erschließbare oder ableitbare Aufgaben durch ein Verfahren wie nachfolgend erläutert.

### Lösung

Ausgehend von dem durch Röhm gesetzten Stand der Technik (DE 102 00 171.5) wurde überraschenderweise gefunden, dass der bevorzugt eingesetzte homogene Katalysator Tetraalkyltitanat (Tetraalkoxytitan) an verschiedenen Stellen des Verfahrens trotz thermischer Beanspruchung in den Kolonnensümpfen und Filmverdampfern immer noch eine beträchtliche Aktivität besitzt. Eine über einen einfachen Durchlauf hinausgehende mehrfache Verwendung des Katalysators ist somit möglich. Hierdurch lassen sich die Hilfsstoffkosten bei unveränderten Produktqualitäten und unveränderten Raum-Zeit- bzw. Gesamt-Ausbeuten deutlich reduzieren. Man benötigt hierzu zusätzlich lediglich einen Flüssigkeitsstromteiler sowie eine Pumpe. Die Steuerung der rückgeführten Katalysatormenge erfolgt anhand des Alkohol (B)- oder MMA (A)-Umsatzes im Reaktionsapparat (1) als Maß für die aktuell vorhandene Katalysatoraktivität. Als weiterer Indikator für die aktuell vorhandene Katalysatoraktivität kann auch die Menge und Zusammensetzung des Niedrigsieder-Kreislaufstroms (14) herangezogen werden.

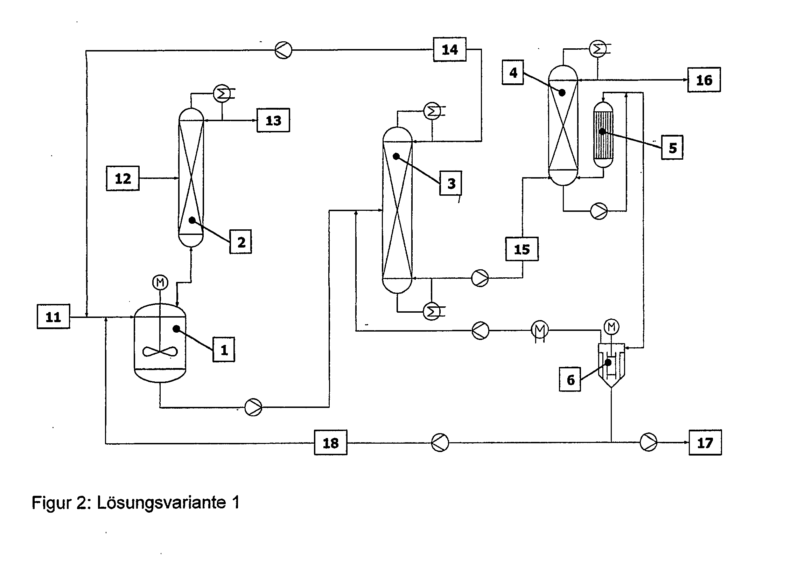

Zur technischen Umsetzung der Katalysatorrezyklierung sind folgende Lösungsvarianten prinzipiell möglich:
Lösungsvariante 1 (Figur 2):
   Der den Katalysator, Polymerisationsinhibitoren sowie hochsiedende Nebenprodukte und restlichen Produktester enthaltende Sumpfabstrom der Vakuum-Eindampfstufe (6) wird geteilt und teilweise in den Reaktionsapparat (1) zurückgeführt (18). Der verbleibende Reststrom wird ausgeschleust (17).
   Die Ausschleusung (17) erfolgt entsprechend der stationär gebildeten Menge an hochsiedenden Nebenkomponenten und entsprechend der verbleibenden Restaktivität des Katalysators im Sumpfablauf der Vakuum-Eindampfstufe (6).
   Die Katalysatorfrischmenge im Strom (11) wird entsprechend der rezyklierten Katalysatormenge gegenüber dem Betrieb ohne Katalysatorrezyklierung reduziert.

Der Rückstrom (18) beträgt zwischen 1 und 95 Gew.-%, bevorzugt zwischen 40 und 90 Gew.-% und ganz besonders bevorzugt zwischen 60 und 85 Gew.-% des Sumpfabstroms aus der Vakuum-Eindampfstufe (6).

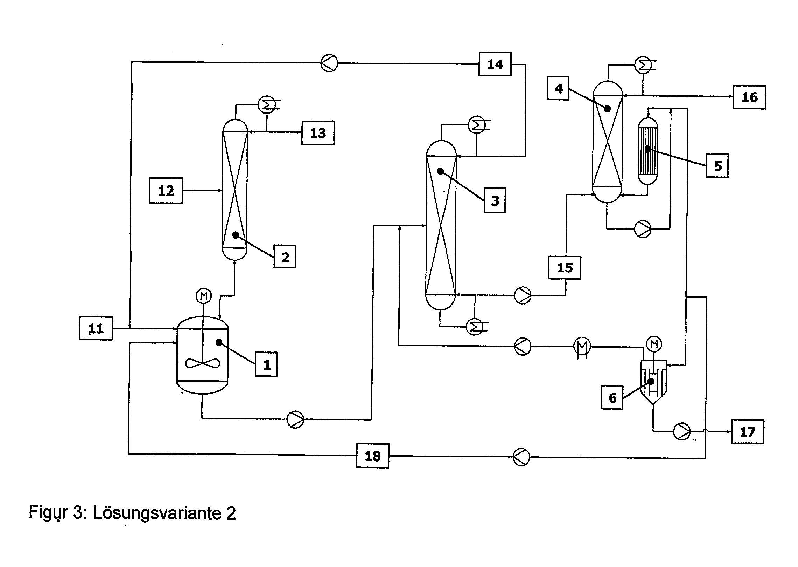

Lösungsvariante 2 (Figur 3):
Der nach Abtrennung des hochreinen Produktesters verbleibende Sumpfabstrom aus dem Apparat mit schonender Filmverdampfung (5), der den Katalysator, Polymerisationsinhibitoren sowie hochsiedende Nebenprodukte und restlichen Produktester enthält, wird geteilt und teilweise in den Reaktionsapparat (1) zurückgeführt (18). Der verbleibende Reststrom wird entsprechend dem Stand der Technik einer Vakuum-Eindampfstufe (6) zugeführt, wo der Großteil des Produktesters abgetrennt und zum Reaktionsapparat (1) oder vorteilhaft zur Niedrigsieder-Destillationskolonne (3) zurückgeführt wird. Der den Katalysator, Polymerisationsinhibitoren sowie hochsiedende Nebenprodukte und restlichen Produktester enthaltende Sumpfabstrom der Vakuum-Eindampfstufe (6) wird ausgeschleust (17). Die Ausschleusung (17) erfolgt entsprechend der stationär gebildeten Menge an hochsiedenden Nebenkomponenten und entsprechend der verbleibenden Restaktivität des Katalysators im Sumpfablauf der Vakuumdestillationsstufe (4, 5). Die Katalysatorfrischmenge im Strom (11) wird entsprechend der rezyklierten Katalysatormenge gegenüber dem Betrieb ohne Katalysatorrezyklierung reduziert.

Der Rückstrom (18) beträgt zwischen 1 und 95 Gew.-%, bevorzugt zwischen 40 und 90 Gew.-% und ganz besonders bevorzugt zwischen 60 und 85 Gew.-% des Sumpfabstroms aus dem Filmverdampfer (5).

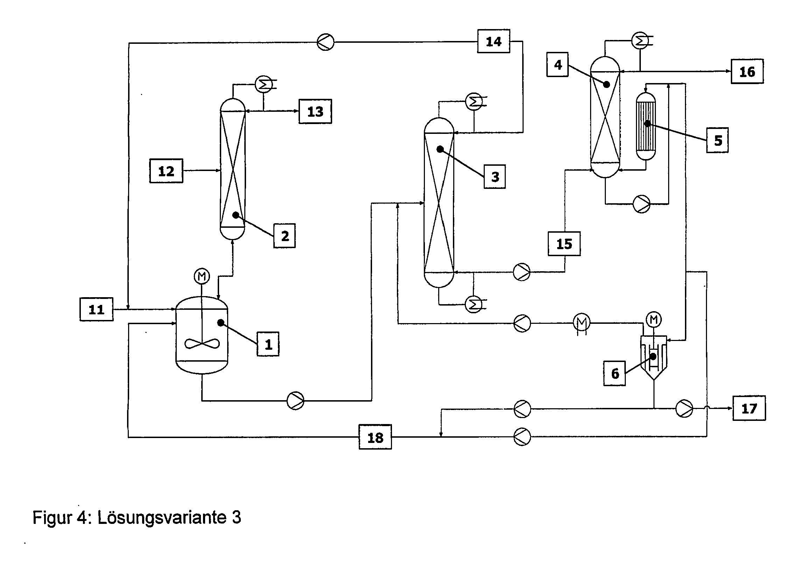

Lösungsvariante 3 (Figur 4):
Der nach Abtrennung des hochreinen Produktesters verbleibende Sumpfabstrom aus dem Apparat mit schonender Filmverdampfung (5), der den Katalysator, Polymerisationsinhibitoren sowie hochsiedende Nebenprodukte und restlichen Produktester enthält, wird geteilt und teilweise in den Reaktionsapparat (1) zurückgeführt (18). Der verbleibende Reststrom wird entsprechend dem Stand der Technik einer Vakuum-Eindampfstufe (6) zugeführt, wo der Großteil des Produktesters abgetrennt und zum Reaktionsapparat (1) oder vorteilhaft zur Niedrigsieder-Destillationskolonne (3) zurückgeführt wird. Der den Katalysator, Polymerisationsinhibitoren sowie hochsiedende Nebenprodukte und restlichen Produktester enthaltende Sumpfabstrom der Vakuum-Eindampfstufe (6) wird geteilt und ebenfalls teilweise in den Reaktionsapparat (1) zurückgeführt (18). Der verbleibende Reststrom wird ausgeschleust (17). Die Ausschleusung (17) erfolgt entsprechend der stationär gebildeten Menge an hochsiedenden Nebenkomponenten und entsprechend der verbleibenden Restaktivität des Katalysators im Recyclestrom (18). Die Katalysatorfrischmenge im Strom (11) wird entsprechend der rezyklierten Katalysatormenge gegenüber dem Betrieb ohne Katalysatorrezyklierung reduziert.

Der Rückstrom (18) beträgt zwischen 1 und 95 Gew.-% bevorzugt zwischen 40 und 90 % Gew.-% und ganz besonders bevorzugt zwischen 60 und 85 Gew.-% der Summe der Sumpfabströme aus dem Filmverdampfer (5) und aus der Vakuum-Eindampfstufe (6).

Beispiele:
Das erfindungsgemäße Verfahren wird durch die nachfolgenden Beispiele näher erläutert, ohne darauf beschränkt zu sein.
Die aufgeführten Beispiele wurden in einer Versuchsanlage im Pilotmaßstab durchgeführt (Stunden-Durchsatz: 6 - 8 kg Feed (MMA und Alkohol) bzw. 5 - 6 kg Produktester). Der Aufbau der Versuchsanlage war wie in Figur 3 dargestellt bzw. in davon leicht abgewandelter Form.

### (Vergleichs-) Beispiel 1: keine Rezyklierung des Katalysators (n-/i-Butylmethacrylatherstellung).

Als Reaktionsapparat (1) wurde ein mit Wasserdampf beheizter Edelstahl-Reaktionskessel mit einem maximalen Füllvolumen von 15 I verwendet. Der Reaktor war über eine Brüdenleitung mit einer oberhalb montierten Azeotrop-Destillationskolonne (2) verbunden. Bei der Azeotrop-Destillationskolonne (Kopfdruck = 1 barabs) handelte es sich um eine mit H = 2 m Sulzer CY Metalldrahtgewebepackungen bestückte Technikums-Glaskolonne mit einem Durchmesser von D = 0,1 m. In der Kolonnenmitte (H = 1 m) befand sich der Zulauf für den Eduktalkohol. Der Reaktorabstrom wurde einer Niedrigsieder-Destillationskolonne (3) kontinuierlich zugeführt. Bei dieser Destillationskolonne handelte es sich um eine mit H = 3,8 m Sulzer CY Metalldrahtgewebepackungen bestückte Technikums-Vakuumglaskolonne (Kopfdruck = 120 mbarabs) mit einem Durchmesser von D = 0,1 m. Der Zulauf befand sich auf H = 2 m. Die Sumpfbeheizung erfolgte mit Wasserdampf. Der kondensierte Kopfaustrag (Kreislaufstrom) (14) wurde kontinuierlich zum Reaktor zurückgeführt. Statt des in Figur 3 dargestellten Fallfilmverdampfers (5) kam bei der kontinuierlichen Aufarbeitung des Sumpfabstroms der Niedrigsieder-Destillationskolonne (15) ein mit Thermalöl beheizter Glasdünnschichtverdampfer mit einer Verdampferfläche von A = 0,1 m² zum Einsatz. Die Brüden dieses Glasdünnschichtverdampfers wurden kontinuierlich in eine oberhalb montierte Hochsieder-Destillationskolonne (4) geleitet. Es handelte sich dabei um eine mit H = 0,5 m Sulzer EX Metalldrahtgewebepackungen bestückte Technikums-Vakuumglaskolonne (Kopfdruck = 120 mbarabs) mit einem Durchmesser von D = 0,05 m. Der Sumpfabstrom wurde einem zweiten, kleineren, ebenfalls mit Thermalöl beheizten Glasdünnschichtverdampfer (6) (Kopfdruck = 120 mbarabs) mit einer Verdampferfläche von A = 0,02 m² kontinuierlich zugeführt. Die Brüden dieses zweiten Glasdünnschichtverdampfers wurden auskondensiert und vereinigt mit dem Reaktorabstrom der Niedrigsieder-Destillationskolonne kontinuierlich zugeführt. Der Sumpfabstrom (17) wurde kontinuierlich aus dem Prozess ausgeschleust. Die Dosierung der Edukte (MMA und Alkohol) erfolgte kontinuierlich mittels Kolben-Dosierpumpen, wobei der Katalysator (Tetraalkyltitanat) im (per Spezifikation wasserfreien) MMA-Feed gelöst zudosiert wurde. Der MMA/Katalysator-Feed wurde dem Reaktor direkt zugeführt, der Alkohol-Feed wurde (auf Kolonneninnentemperatur) vorgewärmt auf die Mitte der Azeotrop-Destillationskolonne aufgegeben. Die kontinuierliche Zugabe von 50 -100 g/h Stabilisatorlösung (0,2 Gew.-% Hydrochinonmonomethylether in MMA bzw. Produktester) erfolgte mit Hilfe von Schlauchpumpen in den Rücklaufstrom der Destillationskolonnen. Die kontinuierliche Förderung der Stoffströme zwischen den Anlagenteilen erfolgte entweder mit Hilfe von Kolben-Dosierpumpen oder durch die Saugwirkung des Vakuums. Zwischenbehälter (Puffervolumina) wurden soweit als möglich vermieden. Die Zusammensetzung der Stoffströme (MMA-, Alkohol-, MeOH- und Produktester-Gehalt) wurde mit Hilfe eines Gaschromatographen bestimmt.

Zur kontinuierlichen Herstellung von n-Butylmethacrylat (n-BuMA) wurden dem Reaktionskessel 4 kg/h MMA-Feed, 18 g/h Tetra-n-Butyltitanat (Ti(n-OBu)₄) und 2,7 kg/h n-BuOH-Feed zudosiert (11). Weiterhin floß dem Reaktor der Kreislaufrückstrom vom Kopf der Niedrigsieder-Destillationskolonne kontinuierlich zu (2,8 kg/h mit folgender Zusammensetzung: 1,0 Gew.-% n-BuMA, 38,3 Gew.-% n-BuOH, 57,3 Gew.-% MMA und 3,4 Gew.-% MeOH). Das molare MMA:n-BuOH-Verhältnis im Reaktorzulauf betrug 1,1:1. Bei einer Reaktorverweilzeit von 1 h und einem MMA/MeOH-Azeotropabzug von 1,5 kg/h stellte sich eine Reaktortemperatur von 115 °C ein. Die Zusammensetzung des MMA/MeOH-Azeotrops betrug 82 Gew.-% MeOH, 18 Gew.-% MMA und < 5 ppm n-BuOH. Der resultierende Reaktorabstrom von 8 kg/h setzte sich wie folgt zusammen: 64,6 Gew.-% n-BuMA, 13,5 Gew.-% n-BuOH, 20,3 Gew.-% MMA, 1,3 Gew.-% MeOH und 0,3 Gew.-% Nebenprodukte. Die Raum-Zeit-Ausbeute des Reaktors bezogen auf n-BuMA betrug somit 570 kg/h/m³. Aufgrund nahezu vollständiger Abtrennung der in bezug auf n-BuMA niedrigsiedenden Komponenten wurde im Sumpfaustrag der Niedrigsieder-Destillationskolonne ein Rohester erhalten (5,8 kg/h), der bereits > 99,5 Gew.-% n-BuMA sowie den gesamten Katalysator und Stabilisator enthielt. Die Ausbeute an n-BuOH bezogen auf den gesamten Prozess betrug folglich nahezu 100 %. Die Ausbeute an MMA bezogen auf den gesamten Prozess betrug abzüglich des zuvor einkalkulierten MMA-Verlustes über das MMA/MeOH-Azeotrop ebenfalls nahezu 100 %. Bei einem Eindampfverhältnis (Verhältnis von Brüden- zu Feedstrom) im ersten, größeren Dünnschichtverdampfer von ca. 90 % werden am Kopf der Hochsieder-Destillationskolonne schließlich 5,1 kg/h Rein-n-BuMA mit folgender Zusammensetzung gewonnen: > 99,92 Gew.-% n-BuMA, < 120 ppm n-BuOH, < 10 ppm MMA, < 5 ppm Di-n-Butylether, Farbzahl (Apha) < 0,2. Bei einem Eindampfverhältnis im zweiten, kleineren Dünnschichtverdampfer von ca. 90 % beträgt die Gesamt-Ausschleusung des Prozesses (Katalysator, Stabilisator, hochsiedende Nebenprodukte, n-BuMA) 0,1 kg/h und der Ausbeuteverlust an n-BuMA bezogen auf das produzierte Rein-n-BuMA < 0,5 Gew.-%.

Der Katalysatorverbrauch beträgt 3,5 g Tetra-n-Butyltitanat / kg Rein-n-BuMA.

Zur kontinuierlichen Herstellung von Iso-Butylmethacrylat (i-BuMA) wurden dem Reaktionskessel 3,4 kg/h MMA-Feed, 19 g/h Tetra-i-Butyltitanat (Ti(i-OBu)₄) und 2,36 kg/h i-BuOH-Feed zudosiert. Weiterhin floß dem Reaktor der Kreislaufrückstrom vom Kopf der Niedrigsieder-Destillationskolonne kontinuierlich zu (2,4 kg/h mit folgender Zusammensetzung: 6,2 Gew.-% i-BuMA, 35,3 Gew.-% i-BuOH, 56,3 Gew.-% MMA und 2,2 Gew.-% MeOH). Das molare MMA:i-BuOH-Verhältnis im Reaktorzulauf betrug 1,1:1. Bei einer Reaktorverweilzeit von 1,2 h und einem MMA/MeOH-Azeotropabzug von 1,26 kg/h stellte sich eine Reaktortemperatur von 115 °C ein. Die Zusammensetzung des MMA/MeOH-Azeotrops betrug 82 Gew.-% MeOH, 18 Gew.-% MMA und < 5 ppm i-BuOH. Der resultierende Reaktorabstrom von 6,9 kg/h setzte sich wie folgt zusammen: 67,3 Gew.-% i-BuMA, 12,0 Gew.-% i-BuOH, 19,4 Gew.-% MMA, 0,8 Gew.-% MeOH und 0,5 Gew.-% Nebenprodukte. Die Raum-Zeit-Ausbeute des Reaktors bezogen auf i-BuMA betrug somit 516 kg/h/m³. Aufgrund nahezu vollständiger Abtrennung der in bezug auf i-BuMA niedrigsiedenden Komponenten wurde im Sumpfaustrag der Niedrigsieder-Destillationskolonne ein Rohester erhalten (5,0 kg/h), der bereits > 99,5 Gew.-% i-BuMA sowie den gesamten Katalysator und Stabilisator enthielt. Die Ausbeute an i-BuOH bezogen auf den gesamten Prozess betrug folglich nahezu 100 %. Die Ausbeute an MMA bezogen auf den gesamten Prozess betrug abzüglich des zuvor einkalkulierten MMA-Verlustes über das MMA/MeOH-Azeotrop ebenfalls nahezu 100 %. Bei einem Eindampfverhältnis (Verhältnis von Brüden- zu Feedstrom) im ersten, größeren Dünnschichtverdampfer von ca. 90 % werden am Kopf der Hochsieder-Destillationskolonne schließlich 4,5 kg/h Rein-i-BuMA mit folgender Zusammensetzung gewonnen: > 99,9 Gew.-% i-BuMA, < 150 ppm i-BuOH, < 10 ppm MMA, 0 ppm Di-i-Butylether, Farbzahl (Apha) < 0,2. Bei einem Eindampfverhältnis im zweiten, kleineren Dünnschichtverdampfer von ca. 90 % beträgt die Gesamt-Ausschleusung des Prozesses (Katalysator, Stabilisator, hochsiedende Nebenprodukte, i-BuMA) 0,05 kg/h und der Ausbeuteverlust an i-BuMA bezogen auf das produzierte Rein-i-BuMA < 0,5 Gew.-%.

Der Katalysatorverbrauch beträgt 4,2 g Tetra-i-Butyltitanat / kg Rein-i-BuMA.

### Beispiel 2: vollständige Rezyklierung des Katalysators (n-Butylmethacrylatherstellung)

Als Reaktionsapparat (1) wurde ein mit Wasserdampf beheizter Edelstahl-Reaktionskessel mit einem maximalen Füllvolumen von 15 I verwendet. Der Reaktor war über eine Brüdenleitung mit einer oberhalb montierten Azeotrop-Destillationskolonne (2) verbunden. Bei der Azeotrop-Destillationskolonne (Kopfdruck = 1 barabs) handelte es sich um eine mit H = 2 m Sulzer CY Metalldrahtgewebepackungen bestückte Technikums-Glaskolonne mit einem Durchmesser von D = 0,1 m. In der Kolonnenmitte (H = 1 m) befand sich der Zulauf für den Eduktalkohol. Der Reaktorabstrom wurde einer Niedrigsieder-Destillationskolonne (3) kontinuierlich zugeführt. Bei dieser Destillationskolonne handelte es sich um eine mit H = 3,8 m Sulzer CY Metalldrahtgewebepackungen bestückte Technikums-Vakuumglaskolonne (Kopfdruck = 120 mbarabs) mit einem Durchmesser von D = 0,1 m. Der Zulauf befand sich auf H = 2 m. Die Sumpfbeheizung erfolgte mit Wasserdampf. Der kondensierte Kopfaustrag (Kreislaufstrom) (14) wurde kontinuierlich zum Reaktor zurückgeführt. Statt des in Figur 3 dargestellten Fallfilmverdampfers (5) kam bei der kontinuierlichen Aufarbeitung des Sumpfabstroms der Niedrigsieder-Destillationskolonne (15) ein mit Thermalöl beheizter Glasdünnschichtverdampfer mit einer Verdampferfläche von A = 0,1 m² zum Einsatz. Die Brüden dieses Glasdünnschichtverdampfers wurden kontinuierlich in eine oberhalb montierte Hochsieder-Destillationskolonne (4) geleitet. Es handelte sich dabei um eine mit H = 0,5 m Sulzer EX Metalldrahtgewebepackungen bestückte Technikums-Vakuumglaskolonne (Kopfdruck = 150 mbarabs) mit einem Durchmesser von D = 0,05 m. Der Sumpfabstrom wurde kontinuierlich in den Reaktionskessel zurückgeführt (18) sowie teilweise aus dem Prozess kontinuierlich ausgeschleust (17). Zum Zeitpunkt t = 0 h wurde der Sumpfabstrom vollständig kontinuierlich in den Reaktionskessel zurückgeführt (18) und es erfolgte keine Ausschleusung (17) sowie keine Frisch-Katalysatordosierung mehr. Die Dosierung der Edukte (MMA und Alkohol) erfolgte kontinuierlich mittels Kolben-Dosierpumpen, wobei der Katalysator (Tetraalkyltitanat) im (per Spezifikation wasserfreien) MMA-Feed gelöst zudosiert wurde. Der MMA/Katalysator-Feed wurde dem Reaktor direkt zugeführt, der Alkohol-Feed wurde (auf Kolonneninnentemperatur) vorgewärmt auf die Mitte der Azeotrop-Destillationskolonne aufgegeben. Die kontinuierliche Zugabe von 50 -100 g/h Stabilisatorlösung (0,2 Gew.-% Hydrochinonmonomethylether in MMA bzw. Produktester) erfolgte mit Hilfe von Schlauchpumpen in den Rücklaufstrom der Destillationskolonnen. Die kontinuierliche Förderung der Stoffströme zwischen den Anlagenteilen erfolgte entweder mit Hilfe von Kolben-Dosierpumpen oder durch die Saugwirkung des Vakuums. Zwischenbehälter (Puffervolumina) wurden soweit als möglich vermieden. Die Zusammensetzung der Stoffströme (MMA-, Alkohol-, MeOH- und Produktester-Gehalt) wurde mit Hilfe eines Gaschromatographen bestimmt.

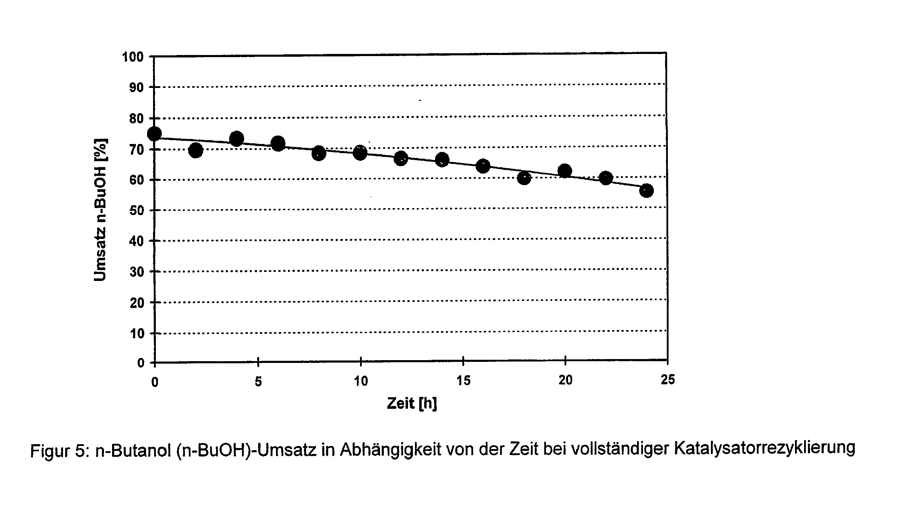

Zur kontinuierlichen Herstellung von n-Butylmethacrylat (n-BuMA) wurden dem Reaktionskessel zunächst 3,8 kg/h MMA-Feed, 18 g/h Tetra-n-Butyltitanat (Ti(n-OBu)4) und 2,7 kg/h n-BuOH-Feed zudosiert. Weiterhin floß dem Reaktor der Kreislaufrückstrom vom Kopf der Niedrigsieder-Destillationskolonne kontinuierlich zu (3,0 kg/h mit folgender Zusammensetzung: 0,2 Gew.-% n-BuMA, 31,1 Gew.-% n-BuOH, 66,4 Gew.-% MMA und 1,9 Gew.-% MeOH). Das molare MMA:n-BuOH-Verhältnis im Reaktorzulauf betrug 1,2:1. Bei einer Reaktorverweilzeit von 1 h und einem MMA/MeOH-Azeotropabzug von 1,4 kg/h stellte sich eine Reaktortemperatur von 115 °C ein. Die Zusammensetzung des MMA/MeOH-Azeotrops betrug 80,4 Gew.-% MeOH, 19,6 Gew.-% MMA und < 5 ppm n-BuOH. Der resultierende Reaktorabstrom von 8,1 kg/h setzte sich wie folgt zusammen: 63,8 Gew.-% n-BuMA, 11,3 Gew.-% n-BuOH, 24,0 Gew.-% MMA, 0,7 Gew.-% MeOH und 0,2 Gew.-% Nebenprodukte. Der n-BuOH-Umsatz des Reaktors betrug somit 74 %. Nachdem zum Zeitpunkt t = 0 keine Ausschleusung (17) und keine Frisch-Katalysatorzufuhr mehr erfolgte, wurde der n-BuOH-Umsatz (als Indikator für die Katalysatoraktivität) über einen Zeitraum von 24 h verfolgt. Wie Figur 5 zu entnehmen ist, nimmt der n-BuOH-Umsatz innerhalb 24 h überraschenderweise nur um 18 % auf 56 % ab, obwohl weder hochsiedende Nebenkomponenten ausgeschleust werden noch frischer Katalysator zugeführt wird und der Katalysator dabei ca. 8 mal durch das gesamte Verfahren umgelaufen ist.

### Beispiel 3: teilweise Rezyklierung des Katalysators (i-Butylmethacrylatherstellung)

Als Reaktionsapparat (1) wurde ein mit Wasserdampf beheizter Edelstahl-Reaktionskessel mit einem maximalen Füllvolumen von 15 I verwendet. Der Reaktor war über eine Brüdenleitung mit einer oberhalb montierten Azeotrop-Destillationskolonne (2) verbunden. Bei der Azeotrop-Destillationskolonne (Kopfdruck = 1 barabs) handelte es sich um eine mit H = 2 m Sulzer CY Metalldrahtgewebepackungen bestückte Technikums-Glaskolonne mit einem Durchmesser von D = 0,1 m. In der Kolonnenmitte (H = 1 m) befand sich der Zulauf für den Eduktalkohol. Der Reaktorabstrom wurde einer Niedrigsieder-Destillationskolonne (3) kontinuierlich zugeführt. Bei dieser Destillationskolonne handelte es sich um eine mit H = 3,8 m Sulzer CY Metalldrahtgewebepackungen bestückte Technikums-Vakuumglaskolonne (Kopfdruck = 120 mbarabs) mit einem Durchmesser von D = 0,1 m. Der Zulauf befand sich auf H = 2 m. Die Sumpfbeheizung erfolgte mit Wasserdampf. Der kondensierte Kopfaustrag (Kreislaufstrom) (14) wurde kontinuierlich zum Reaktor zurückgeführt. Statt des in Figur 3 dargestellten Fallfilmverdampfers (5) kam bei der kontinuierlichen Aufarbeitung des Sumpfabstroms der Niedrigsieder-Destillationskolonne (15) ein mit Thermalöl beheizter Glasdünnschichtverdampfer mit einer Verdampferfläche von A = 0,1 m² zum Einsatz. Die Brüden dieses Glasdünnschichtverdampfers wurden kontinuierlich in eine oberhalb montierte Hochsieder-Destillationskolonne (4) geleitet. Es handelte sich dabei um eine mit H = 0,5 m Sulzer EX Metalldrahtgewebepackungen bestückte Technikums-Vakuumglaskolonne (Kopfdruck = 150 mbarabs) mit einem Durchmesser von D = 0,05 m. Der Sumpfabstrom wurde geteilt und teilweise kontinuierlich in den Reaktionskessel zurückgeführt (18) sowie teilweise aus dem Prozess kontinuierlich ausgeschleust (17). Die Dosierung der Edukte (MMA und Alkohol) erfolgte kontinuierlich mittels Kolben-Dosierpumpen, wobei der Katalysator (Tetraalkyltitanat) im (per Spezifikation wasserfreien) MMA-Feed gelöst zudosiert wurde. Der MMA/Katalysator-Feed wurde dem Reaktor direkt zugeführt, der Alkohol-Feed wurde (auf Kolonneninnentemperatur) vorgewärmt auf die Mitte der Azeotrop-Destillationskolonne aufgegeben. Die kontinuierliche Zugabe von 50 -100 g/h Stabilisatorlösung (0,2 Gew.-% Hydrochinonmonomethylether in MMA bzw. Produktester) erfolgte mit Hilfe von Schlauchpumpen in den Rücklaufstrom der Destillationskolonnen. Die kontinuierliche Förderung der Stoffströme zwischen den Anlagenteilen erfolgte entweder mit Hilfe von Kolben-Dosierpumpen oder durch die Saugwirkung des Vakuums. Zwischenbehälter (Puffervolumina) wurden soweit als möglich vermieden. Die Zusammensetzung der Stoffströme (MMA-, Alkohol-, MeOH- und Produktester-Gehalt) wurde mit Hilfe eines Gaschromatographen bestimmt.

Zur kontinuierlichen Herstellung von Iso-Butylmethacrylat (i-BuMA) wurden dem Reaktionskessel 2,78 kg/h MMA-Feed, 3 g/h Tetra-i-Butyltitanat (Ti(i-OBu)₄) und 1,9 kg/h i-BuOH-Feed zudosiert. Weiterhin floß dem Reaktor der Kreislaufrückstrom vom Kopf der Niedrigsieder-Destillationskolonne kontinuierlich zu (2,2 kg/h mit folgender Zusammensetzung: 2,8 Gew.-% i-BuMA, 39,3 Gew.-% i-BuOH, 56,2 Gew.-% MMA und 1,7 Gew.-% MeOH). Das molare MMA:i-BuOH-Verhältnis im Reaktorzulauf betrug 1,1:1. Bei einer Reaktorverweilzeit von 1,2 h und einem MMA/MeOH-Azeotropabzug von 1,04 kg/h stellte sich eine Reaktortemperatur von 116 °C ein. Die Zusammensetzung des MMA/MeOH-Azeotrops betrug 79 Gew.-% MeOH, 21 Gew.-% MMA und < 5 ppm i-BuOH. Der resultierende Reaktorabstrom von 6,5 kg/h setzte sich wie folgt zusammen: 66,8 Gew.-% i-BuMA, 13,1 Gew.-% i-BuOH, 19,0 Gew.-% MMA, 0,5 Gew.-% MeOH und 0,6 Gew.-% Nebenprodukte. Die Raum-Zeit-Ausbeute des Reaktors bezogen auf i-BuMA betrug somit 417 kg/h/m³. Aufgrund nahezu vollständiger Abtrennung der in bezug auf i-BuMA niedrigsiedenden Komponenten wurde im Sumpfaustrag der Niedrigsieder-Destillationskolonne ein Rohester erhalten (4,25 kg/h), der bereits > 99,5 Gew.-% i-BuMA sowie den gesamten Katalysator und Stabilisator enthielt. Die Ausbeute an i-BuOH bezogen auf den gesamten Prozess betrug folglich nahezu 100 %. Die Ausbeute an MMA bezogen auf den gesamten Prozess betrug abzüglich des zuvor einkalkulierten MMA-Verlustes über das MMA/MeOH-Azeotrop ebenfalls nahezu 100 %. Bei einem Eindampfverhältnis (Verhältnis von Brüden- zu Feedstrom) im Dünnschichtverdampfer von ca. 90 % werden am Kopf der Hochsieder-Destillationskolonne schließlich 3,81 kg/h Rein-i-BuMA mit folgender Zusammensetzung gewonnen: > 99,9 Gew.-% i-BuMA, < 150 ppm i-BuOH, < 10 ppm MMA, 0 ppm Di-i-Butylether, Farbzahl (Apha) < 0,2. Bei einer 10 prozentigen Ausschleusung (0,05 kg/h) bzw. einer 90 prozentigen Rückführung (0,45 kg/h) des Sumpfabstroms des Dünnschichtverdampfers beträgt der Katalysatorverbrauch 0,8 g Tetra-i-Butyltitanat / kg Rein-i-BuMA.

Gegenüber Beispiel 1 beträgt die Katalysatoreinsparung somit ca. 80 %.

### Erläuterung der Bezugszeichen:

- 1: Reaktionsapparat
- 2: Azeotrop-Destillationskolonne
- 3: Niedrigsieder-Destillationskolonne
- 4: Hochsieder-Destillationskolonne
- 5: Filmverdampfer
- 6: Filmverdampfer
- 11: Methyl(meth)acrylat- und Katalysator-Feed
- 12: Alkohol-Feed
- 13: Methanol/Methyl(meth)acrylat-Azeotrop
- 14: Niedrigsieder-Kreislaufstrom
- 15: Roh-Ester
- 16: Rein-Ester
- 17: Hochsieder und Katalysator
- 18: Katalysatorrezyktierung

## Patentansprüche

1. Verfahren zur kontinuierlichen Herstellung von höheren (Meth)acrylsäureestern (C) durch Umesterung von (Meth)acrylsäuremethylester (A) mit höheren Alkoholen (B) in Gegenwart eines Katalysators oder Katalysatorgemisches,
**dadurch gekennzeichnet,**
**dass** der Sumpfabstrom der Vakuum-Eindampfstufe (6) geteilt wird und teilweise dem Reaktionsapparat (1) zugeführt wird, wobei unter höheren Alkoholen Alkohole mit 2 bis 12 C-Atomen verstanden werden.

2. Verfahren nach Anspruch 1 zur kontinuierlichen Herstellung von höheren (Meth)acrylsäureestern (C) durch Umesterung von (Meth)acrylsäuremethylester (A) mit höheren Alkoholen (B) in Gegenwart eines Katalysators oder Katalysatorgemisches,
**dadurch gekennzeichnet,**
**dass** der Sumpfabstrom aus dem Filmverdampfer (5) geteilt wird und teilweise dem Reaktionsapparat (1) zugeführt wird.

3. Verfahren nach Anspruch 1 zur kontinuierlichen Herstellung von höheren (Meth)acrylsäureestern (C) durch Umesterung von (Meth)acrylsäuremethylester (A) mit höheren Alkoholen (B) in Gegenwart eines Katalysators oder Katalysatorgemisches,
**dadurch gekennzeichnet,**
**dass** der Sumpfabstrom aus dem Filmverdampfer (5) geteilt wird und teilweise dem Reaktionsapparat (1) zugeführt wird und dass der Sumpfabstrom der Vakuum-Eindampfstufe (6) geteilt wird und dem Reaktionsapparat (1) zugeführt wird.

4. Verfahren nach Anspruch 1, 2 oder 3,
**dadurch gekennzeichnet,**
**dass** als Alkohole n-Butanol, iso-Butanol oder 2-Ethylhexanol verwendet werden.

5. Verfahren nach Anspruch 1, 2 oder 3,
**dadurch gekennzeichnet,**
**dass** als Katalysator homogene Katalysatoren verwendet werden.

6. Verfahren nach Anspruch 5,
**dadurch gekennzeichnet,**
**dass** als Katalysator das Titanat des Alkohols (B) eingesetzt wird.

7. Verfahren nach Anspruch 1,
**dadurch gekennzeichnet,**
**dass** 1 - 95 Gew.-% des Sumpfabstroms aus der Vakuum-Eindampfstufe (6) dem Reaktionsapparat zugeführt werden.

8. Verfahren nach Anspruch 7,
**dadurch gekennzeichnet,**
**dass** 40 - 90 Gew.-% des Sumpfabstroms aus der Vakuum-Eindampfstufe (6) dem Reaktionsapparat (1) zugeführt werden.

9. Verfahren nach Anspruch 8,
**dadurch gekennzeichnet,**
**dass** 60 - 85 Gew.-% des Sumpfabstroms aus der Vakuum-Eindampfstufe (6) dem Reaktionsapparat (1) zugeführt werden.

10. Verfahren nach Anspruch 2,
**dadurch gekennzeichnet,**
**dass** 1 - 95 Gew.-% des Sumpfabstroms aus dem Filmverdampfer (5) dem Reaktionsapparat (1) zugeführt werden.

11. Verfahren nach Anspruch 10,
**dadurch gekennzeichnet,**
**dass** 40 - 90 Gew.-% des Sumpfabstroms aus dem Filmverdampfer (5) dem Reaktionsapparat (1) zugeführt werden.

12. Verfahren nach Anspruch 11,
**dadurch gekennzeichnet,**
**dass** 60 - 85 Gew.-% des Sumpfabstroms aus dem Filmverdampfer (5) dem Reaktionsapparat (1) zugeführt werden.

13. Verfahren nach Anspruch 3,
**dadurch gekennzeichnet,**
**dass** 1 - 95 Gew.-% der Summe der Sumpfabströme aus dem Filmverdampfer (5) und aus der Vakuum-Eindampfstufe (6) dem Reaktionsapparat (1) zugeführt werden.

14. Verfahren nach Anspruch 13,
**dadurch gekennzeichnet,**
**dass** 40 - 90 % der Summe der Sumpfabströme aus dem Filmverdampfer (5) und aus der Vakuum-Eindampfstufe (6) dem Reaktionsapparat (1) zugeführt werden.

15. Verfahren nach Anspruch 14,
**dadurch gekennzeichnet,**
**dass** 60 - 85 Gew.-% der Summe der Sumpfabströme aus dem Filmverdampfer (5) und aus der Vakuum-Eindampfstufe (6) dem Reaktionsapparat (1) zugeführt werden.

## Claims

1. Process for continuously preparing higher (meth)acrylic esters (C) by transesterifying methyl (meth)acrylate (A) with higher alcohols (B) in the presence of a catalyst or catalyst mixture,
**characterized in that**
the bottom effluent of the vacuum evaporation stage (6) is divided and is fed in part to the reaction apparatus (1), higher alcohols being understood to mean alcohols having 2 to 12 carbon atoms.

2. Process according to Claim 1 for continuously preparing higher (meth)acrylic esters (C) by transesterifying methyl (meth)acrylate (A) with higher alcohols (B) in the presence of a catalyst or catalyst mixture,
**characterized in that**
the bottom effluent from the film evaporator (5) is divided and is fed in part to the reaction apparatus (1).

3. Process according to Claim 1 for continuously preparing higher (meth)acrylic esters (C) by transesterifying methyl (meth)acrylate (A) with higher alcohols (B) in the presence of a catalyst or catalyst mixture,
**characterized in that**
the bottom effluent from the film evaporator (5) is divided and is fed in part to the reaction apparatus (1) and **in that** the bottom effluent of the vacuum evaporation stage (6) is divided and is fed to the reaction apparatus (1).

4. Process according to Claim 1, 2 or 3,
**characterized in that**
the alcohols used are n-butanol, isobutanol or 2-ethylhexanol.

5. Process according to Claim 1, 2 or 3,
**characterized in that**
the catalyst used is a homogeneous catalyst.

6. Process according to Claim 5,
**characterized in that**
the catalyst used is the titanate of the alcohol (B).

7. Process according to Claim 1,
**characterized in that**
1 - 95% by weight of the bottom effluent from the vacuum evaporation stage (6) is fed to the reaction apparatus.

8. Process according to Claim 7,
**characterized in that**
40 - 90% by weight of the bottom effluent from the vacuum evaporation stage (6) is fed to the reaction apparatus (1).

9. Process according to Claim 8,
**characterized in that**
60 - 85% by weight of the bottom effluent from the vacuum evaporation stage (6) is fed to the reaction apparatus (1).

10. Process according to Claim 2,
**characterized in that**
1 - 95% by weight of the bottom effluent from the film evaporator (5) is fed to the reaction apparatus (1).

11. Process according to Claim 10,
**characterized in that**
40 - 90% by weight of the bottom effluent from the film evaporator (5) is fed to the reaction apparatus (1).

12. Process according to Claim 11,
**characterized in that**
60 - 85% by weight of the bottom effluent from the film evaporator (5) is fed to the reaction apparatus (1).

13. Process according to Claim 3,
**characterized in that**
1 - 95% by weight of the sum of the bottom effluents from the film evaporator (5) and from the vacuum evaporation stage (6) is fed to the reaction apparatus (1).

14. Process according to Claim 13,
**characterized in that**
40 - 90% [lacuna] of the sum of the bottom effluents from the film evaporator (5) and from the vacuum evaporation stage (6) is fed to the reaction apparatus (1).

15. Process according to Claim 14,
**characterized in that**
60 - 85% by weight of the sum of the bottom effluents from the film evaporator (5) and from the vacuum evaporation stage (6) is fed to the reaction apparatus (1).

## Revendications

1. Procédé pour la production en continu d'esters supérieurs d'acide (méth)acrylique (C) par transestérification de (méth)acrylate de méthyle (A) avec des alcools supérieurs (B) en présence d'un catalyseur ou mélange de catalyseurs,
**caractérisé en ce que**
le courant de décharge de fond de l'étage d'évaporation sous vide (6) est divisé et en partie envoyé à l'appareil de réaction (1), en entendant par alcools supérieurs des alcools ayant de 2 à 12 atomes de carbone.

2. Procédé selon la revendication 1 pour la production en continu d'esters supérieurs d'acide (méth)acrylique (C) par transestérification de (méth)acrylate de méthyle (A) avec des alcools supérieurs (B) en présence d'un catalyseur ou mélange de catalyseurs,
**caractérisé en ce que**
le courant de décharge de fond provenant de l'évaporateur à film (5) est divisé et en partie envoyé à l'appareil de réaction (1).

3. Procédé selon la revendication 1 pour la production en continu d'esters supérieurs d'acide (méth)acrylique (C) par transestérification de (méth)acrylate de méthyle (A) avec des alcools supérieurs (B) en présence d'un catalyseur ou mélange de catalyseurs,
**caractérisé en ce que**
le courant de décharge de fond provenant de l'évaporateur à film (5) est divisé et en partie envoyé à l'appareil de réaction (1) et **en ce que** le courant de décharge de fond de l'étage d'évaporation sous vide (6) est divisé et envoyé à l'appareil de réaction (1).

4. Procédé selon la revendication 1, 2 ou 3,
**caractérisé**
**en ce qu'**on utilise comme alcools le n-butanol, l'isobutanol ou le 2-éthylhexanol.

5. Procédé selon la revendication 1, 2 ou 3,
**caractérisé**
**en ce qu'**on utilise comme catalyseur des catalyseurs homogènes.

6. Procédé selon la revendication 5,
**caractérisé**
**en ce qu'**on utilise comme catalyseur le titanate de l'alcool (B).

7. Procédé selon la revendication 1,
**caractérisé en ce que**
1 - 95 % en poids du courant de décharge de fond provenant de l'étage d'évaporation sous vide (6) sont envoyés à l'appareil de réaction.

8. Procédé selon la revendication 7,
**caractérisé en ce que**
40 - 90 % en poids du courant de décharge de fond provenant de l'étage d'évaporation sous vide (6) sont envoyés à l'appareil de réaction (1).

9. Procédé selon la revendication 8,
**caractérisé en ce que**
60 - 85 % en poids du courant de décharge de fond provenant de l'étage d'évaporation sous vide (6) sont envoyés à l'appareil de réaction (1).

10. Procédé selon la revendication 2,
**caractérisé en ce que**
1 - 95 % en poids du courant de décharge de fond provenant de l'évaporateur à film (5) sont envoyés à l'appareil de réaction (1).

11. Procédé selon la revendication 10,
**caractérisé en ce que**
40 - 90 % en poids du courant de décharge de fond provenant de l'évaporateur à film (5) sont envoyés à l'appareil de réaction (1).

12. Procédé selon la revendication 11,
**caractérisé en ce que**
60 - 85 % en poids du courant de décharge de fond provenant de l'évaporateur à film (5) sont envoyés à l'appareil de réaction (1).

13. Procédé selon la revendication 3,
**caractérisé en ce que**
1 - 95 % en poids de la somme des courants de décharge de fond provenant de l'évaporateur à film (5) et de l'étage d'évaporation sous vide (6) sont envoyés à l'appareil de réaction (1).

14. Procédé selon la revendication 13,
**caractérisé en ce que**
40 - 90 % en poids de la somme des courants de décharge de fond provenant de l'évaporateur à film (5) et de l'étage d'évaporation sous vide (6) sont envoyés à l'appareil de réaction (1).

15. Procédé selon la revendication 14,
**caractérisé en ce que**
60 - 85 % en poids de la somme des courants de décharge de fond provenant de l'évaporateur à film (5) et de l'étage d'évaporation sous vide (6) sont envoyés à l'appareil de réaction (1).
